# EUROPEAN PATENT APPLICATION

(11) **EP 3 950 037 A1**
(43) Date of publication of application: **09.02.2022**
(21) Application number: 19922821.4
(22) Date of filing: 05.04.2019
(51) Int. Cl.: A61M 25/092, A61M 25/01

(54) **CATHETER HANDLE AND TIP-DEFLECTABLE STEERABLE CATHETER**

(71) Applicant: Japan Lifeline Co., Ltd., Tokyo 140-0002 (JP)
(72) Inventor: WATANABE Takafumi, Tokyo 140-0002 (JP)
(74) Representative: Grünecker Patent- und Rechtsanwälte PartG mbB
(86) International application number: PCT/JP2019/015121
(87) International publication number: WO 2020/202546

(57) **Abstract**

An object is to provide a handle that can sufficiently elongate the pull amount of an operation wire and that can insert a tube component that extends out from a lumen of a catheter shaft to the inside of the handle body without allowing the tube component to contact the rotary plate and the operation wire. A handle (100) according to the present invention includes a handle body (10) that is composed of a first handle member (11) and a second handle member (12), and a rotational operation unit (20); the rotational operation unit includes a rotary plate (25), a first spacer member (21) non-rotatably attached to the handle body, and a second spacer member (22) non-rotatably attached to the handle body; the rotary plate includes a guide rail (251) that defines paths of operation wires (501, 502) in the rotational operation unit so that the paths detour along a circumferential direction of the rotary plate; and in the first spacer member and the second spacer member, respectively, guide paths (211, 221) of tube components are formed.

## Description

### Technical Field

The present invention relates to a catheter handle and a distal-end-deflectable catheter.

### Background Art

A medical catheter is usually formed by attaching a handle to the proximal end side of a catheter shaft. As the catheter handle, various handles have been proposed in accordance with the types of catheters. For example, there may be a case where a user wants to bend and stretch a distal end portion of the catheter by operating the catheter handle. As a mechanism for deflecting the distal end portion of the catheter by manipulation on the proximal side, a mechanism in which a proximal end of an operation wire is coupled to a rotary plate, which is rotatably attached to a handle body, and that changes (deflects) the direction of the distal end of the catheter by rotationally operating the rotary plate has been introduced (see PTL 1).

The present applicant has proposed a catheter handle that can insert, to the inside of a handle body, an elongated component extended out from a lumen of a catheter shaft, such as a conductive wire of an electrode and various tubes, without allowing the elongated component to contact a rotary plate (see PTL 2).

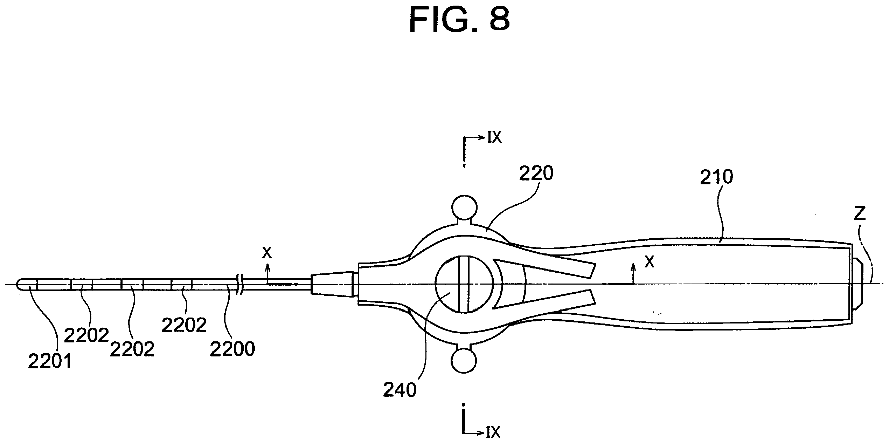

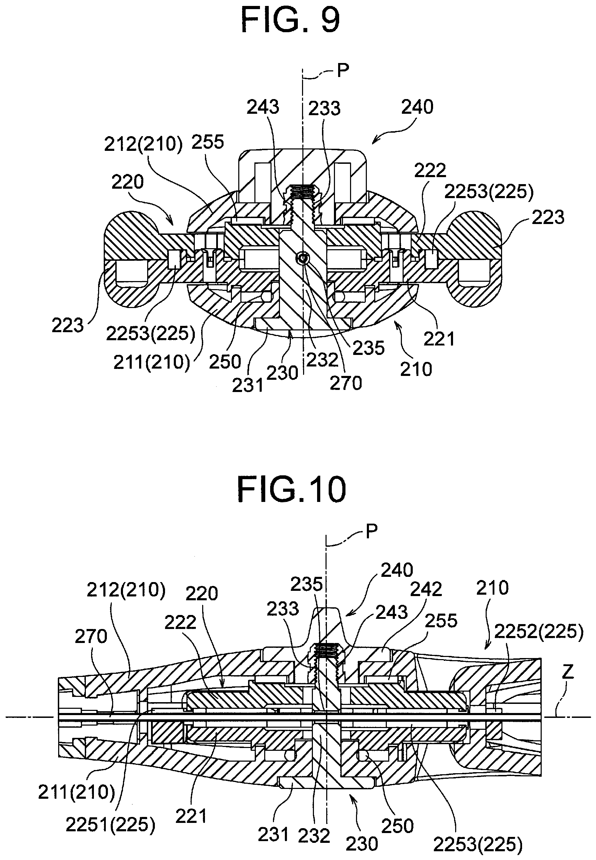

The catheter handle described in PTL 2 is a handle that is to be attached to a proximal end side of a catheter shaft in order to constitute an electrode catheter. As illustrated in Figs. 8 to 10, the catheter handle includes: a handle body 210 that is formed by combining a first handle member 211 and a second handle member 212; a rotational operation unit including a rotary plate 220 that is disposed between the first handle member 211 and the second handle member 212, that is attached to the handle body 210 so as to be rotatable around a rotation axis (P), and to which a proximal end of an operation wire for deflecting a distal end of the catheter shaft is coupled; an adjustment pin 230 whose base portion 231 is fixed to the first handle member 211, whose shaft portion 232 is disposed so as to extend through the rotary plate 220 along the rotation axis (P), and that includes a male thread portion 233 on the distal end side thereof; an adjustment knob 240 that includes a female thread portion 243 threadedly engaged with the male thread portion 233 of the adjustment pin 230 and that is rotatably attached to the second handle member 212; an O-ring 250 that is disposed between the first handle member 211 and the rotary plate 220 so that the operational force of the rotary plate 220 changes in accordance with the threaded engagement depth of the male thread portion 233 of the adjustment pin 230 and the female thread portion 243 of the adjustment knob 240; and a washer 255 that is disposed between the second handle member 212 and the rotary plate 220. The rotational operation unit includes the rotary plate 220 that is formed by combining a first rotation member 221 and a second rotation member 222, and an intermediate member 225 that is disposed between the first rotation member 221 and the second rotation member 222 of the rotary plate 220 and that is non-rotatably attached to the handle body 210. In the intermediate member 225, guide grooves (a guide groove 2251 on the distal end side and a guide groove 2252 on the proximal end side), for enabling a tube 270 (elongated component) inserted to the inside of the handle body 210 to extend along a central axis (Z) of the handle body 210 in the longitudinal direction, and an outer peripheral wall 2253 of the rotational operation unit, which has a shape coinciding with the outer peripheral shape of the rotary plate 220, are integrally formed. In the shaft portion 232 of the adjustment pin 230, a through hole 235, through which the tube 270 extending along the central axis (Z) can be inserted, is formed. In Fig. 8, 2200 denotes a catheter shaft, 2201 denotes a distal end electrode, 2202 denotes a ring-shaped electrode; and, in Fig. 9, 223 denotes the knob of the rotary plate 220.

With the catheter handle, by pulling the operation wire by rotating the rotary plate 220, it is possible to bend the distal end portion of the catheter shaft and to deflect the distal end of the catheter shaft. Because the intermediate member 225 is disposed between the first rotation member 221 and the second rotation member 222 of the rotary plate 220, the intermediate member 225 functions as a spacer, and it is possible to reliably provide an insertion space for the tube 270 (elongated component) between the first rotation member 221 and the second rotation member 222.

However, the catheter handle described in PTL 2 has a problem in that, because it is not possible to reliably provide a sufficient pull amount by which the operation wire is pulled by rotating the rotary plate 220, it is not possible to sufficiently bend the distal end portion of the catheter shaft 2200 (defect the distal end).

On the other hand, the present applicant has proposed a catheter handle including a mechanism for reliably providing a sufficient pull amount by which the operation wire is pulled by rotating the rotary plate (see PTL 3).

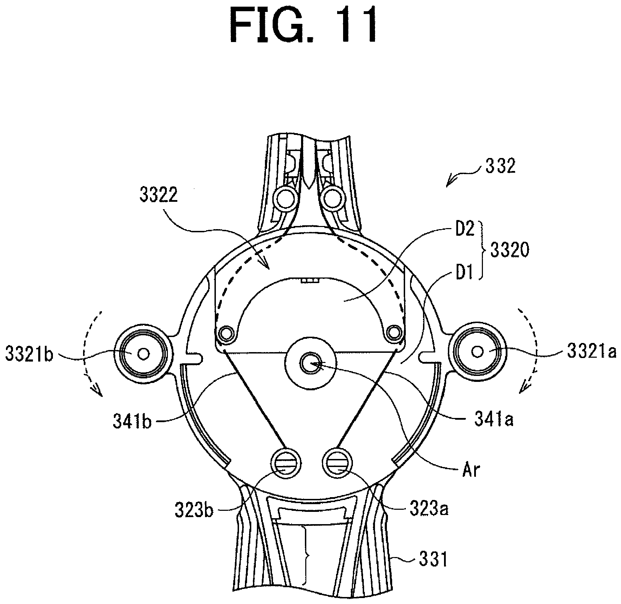

The catheter handle described in PTL 3 is a handle to be attached to a proximal end side of a catheter shaft. As illustrated in Fig. 11, the catheter handle includes a handle body 331 and a rotational operation unit 332 including a rotary plate 3320 that is rotatably attached to the handle body 331. The rotary plate 3320 includes a body portion D1 that is operated during a rotational operation and an attachment member D2 that is attachable to and detachable from the body portion D1 and that includes a guide mechanism (guide rail 3322) that defines the paths of an operation wire 341a and an operation wire 341b for bending the distal end portion of the catheter shaft in accordance with the rotational operation. In Fig. 11, 3321a and 3321b denote knobs of the rotary plate 3320, 323a and 323b respectively denote anchors for fixing the proximal ends of the operation wire 341a and the operation wire 341b to the rotary plate 3320, and Ar denotes the rotation axis of the rotary plate 3320.

With the catheter handle, by rotating the rotary plate 3320 clockwise by operating the knob 3321a, the operation wire 341a is pulled toward the proximal end side in the inside of the catheter shaft and the handle body 331, and the distal end portion of the catheter shaft bends in the first direction. At this time, because the operation wire 341a in the rotational operation unit 332 is guided by the guide rail 3322 and moves in the vicinity of the circumference of the rotary plate 3320, it is possible to reliably provide a sufficient pull amount of the operation wire 341a.

On the other hand, by rotating the rotary plate 3320 counterclockwise by operating the knob 3321b, the operation wire 341b is pulled toward the proximal end side in the inside of the catheter shaft and the handle body 331, and the distal end portion of the catheter shaft bends in a second direction opposite to the first direction. At this time, because the operation wire 341b in the rotational operation unit 332 is guided by the guide rail 3322 and moves in the vicinity of the circumference of the rotary plate 3320, it is possible to reliably provide a sufficient pull amount of the operation wire 341b.

In an electrode catheter including the catheter handle described in PTL 3, a conductive wire of an electrode (a distal end electrode and/or a ring-shaped electrode) attached to catheter shaft passes through the lumen of the catheter shaft and extends out from the proximal end opening of the lumen, passes through a very small gap between the handle body 331 and the rotary plate 3320 in the rotational operation unit 332, and is inserted to the inside of the handle body 331.

However, with the catheter handle described in PTL 3, it is substantially impossible to insert, to the inside of the handle body, a tube component, such as a flow tube for supplying a fluid to the lumen of the catheter shaft, that extends out from the proximal end opening of the lumen of the catheter shaft.

That is, due to the structure of the rotary plate 3320 having the guide mechanism (guide rail 3322) for guiding the operation wire, it is not possible to reliably provide a space for inserting a tube component to the inside of the rotary plate 3320 as in the catheter handle described in PTL 2. Therefore, in order to insert a tube component that extends out from the proximal end opening of the lumen of the catheter shaft to the inside of the handle body 331, it is necessary to pass the tube component through the gap between the handle body 331 and the rotary plate 3320.

However, in the catheter handle described in PTL 3, a gap through which a tube component can be passed is not reliably provided between the handle body 331 and the rotary plate 3320. Even if a gap through which a tube component can be passed is reliably provided between the handle body 331 and the rotary plate 3320, in a case where a tube component that extends out from a lumen that opens on the other side of the rotary plate 3320 is passed through the gap between the handle body 331 and one side of the rotary plate 3320, the tube component may kink due to the height difference, and the flow performance of a fluid may be impaired or the tube component may break. Moreover, in such a case, a tube component that extends from the other side toward the one side of the rotary plate 3320 may contact the rotary plate 3320 or the operation wires 341a and 341b and may become damaged.

### Citation List

### Patent Literature

PTL1: Japanese Unexamined Patent Application Publication No. 2005-230471
PTL 2: Japanese Patent No. 5535260
PTL 3: Japanese Unexamined Patent Application Publication No. 2018-153460

### Summary of Invention

### Technical Problem

The present invention has been made based on such circumstances, and an object thereof is to provide a catheter handle that can sufficiently elongate the pull amount by which an operation wire is pulled by rotating a rotary plate and that can cause a tube component that extends out from a proximal end of a catheter shaft to be inserted to the inside of a handle body without allowing the tube component to contact the rotary plate and the operation wire.

### Solution to Problem

(1) A catheter handle according to the present invention is a catheter handle to be attached to a proximal end side of a catheter shaft.

The catheter handle includes: a handle body that is formed by combining a first handle member and a second handle member that are divided into two along a longitudinal direction; and a rotational operation unit that is disposed between the first handle member and the second handle member, that includes a rotary plate that is disc-shaped and that is attached to the handle body so as to be rotatable around a rotation axis perpendicular to the longitudinal direction, and to which each proximal end of a first operation wire and a second operation wire for respectively deflecting a distal end of the catheter shaft in a first direction and in a second direction is coupled.

The rotational operation unit includes the rotary plate,
a first spacer member that is disposed between the rotary plate and the first handle member, that is non-rotatably attached to the handle body, and that includes an outer peripheral wall having a shape coinciding with an outer peripheral shape of the rotary plate, and
a second spacer member that is disposed between the rotary plate and the second handle member, that is non-rotatably attached to the handle body, and that includes an outer peripheral wall having a shape coinciding with the outer peripheral shape of the rotary plate.

The rotary plate includes a guide mechanism that defines paths of the first operation wire and the second operation wire in the rotational operation unit so that the paths detour along a circumferential direction of the rotary plate.

In the first spacer member and the second spacer member, respectively, guide paths for inserting tube components extending out from a proximal end of the catheter shaft to an inside of the handle body so that the tube components do not contact the rotary plate (so that the tube components are separated from both surfaces of the rotary plate) are formed.

The rotary plate need not be perfectly disc-shaped, and may be, for example, elliptical-disc-shaped.

With the catheter handle having such a structure, because the rotary plate thereof includes the guide mechanism that defines the paths of the first operation wire and the second operation wire in the rotational operation unit so that the paths detour along the circumferential direction of the rotary plate, it is possible to sufficiently elongate the pull amount by which the first operation wire and the second operation wire are pulled by rotating the rotary plate.

With the first spacer member, it is possible to reliably provide an insertion space for the tube component between the rotary plate and the first handle member; with the second spacer member, it is possible to reliably provide an insertion space for the tube component between the rotary plate and the second handle member; and, by passing the tube components, which extend out from the proximal end of the catheter shaft, through the guide path formed in the first spacer member or the second spacer member, it is possible to insert the tube components to the inside of the handle body without allowing the tube components to contact the rotary plate. Because contact of the tube components with the rotary plate can be avoided, it is possible to reliably prevent generation of noise, damage to (abrasion of) the tube components, and the like during the operation of the rotary plate.

Moreover, when the catheter shaft has a multi-lumen structure, by passing the tube component extending out from the proximal end opening of the lumen that is formed in a semi-circumference part of the catheter shaft on a side on which the first handle member is positioned through the guide path formed in the first spacer member and by passing the tube component extending out from the proximal end opening of the lumen that is formed in a semi-circumference part of the catheter shaft on a side on which the second handle member is positioned through the guide path formed in the second spacer member, it is possible to insert the tube components to the inside of the handle body without allowing the tube components to contact the operation wires.

(2) In the catheter handle according to the present invention, preferably, the catheter handle includes:
an adjustment pin including a base portion that is fixed to the first handle member, a shaft portion that is disposed so as to extend through the rotary plate along the rotation axis of the rotary plate, and a male thread portion that is formed on a distal end side of the shaft portion,
an adjustment knob that includes a female thread portion threadedly engaged with the male thread portion of the adjustment pin and that is rotatably attached to the second handle member; and
an elastic member that is disposed between the handle body and the rotary plate so that an operational force of the rotary plate changes in accordance with a threaded engagement depth of the male thread portion of the adjustment pin and the female thread portion of the adjustment knob; and,
in the shaft portion of the adjustment pin, a through-hole or a cutout through which each of the tube components is insertable is formed.

With the catheter handle having such a structure, it is not necessary to detour the tube components, which extends out from the proximal end of the catheter shaft, around the shaft portion of the adjustment pin, and it is possible to cause the tube components to extend to the inside of the handle body along the longitudinal direction of the handle body.

(3) In the catheter handle according to the present invention, preferably, the guide paths that are respectively formed in the first spacer member and the second spacer member are guide grooves that are integrally formed with the outer peripheral walls and that have depressions on the rotary plate side.

(4) In the catheter handle according to the present invention, preferably, at least one of the tube components is a flow tube through which a fluid is to flow.

(5) In the catheter handle according to the present invention, preferably, at least one of the tube components is a protective tube that encloses a conductive wire and/or another component.

(6) In the catheter handle according to the present invention, preferably, the rotational operation unit includes a first coupling portion to which the proximal end of the first operation wire is coupled and a second coupling portion to which the proximal end of the second operation wire is coupled, and
the first coupling portion and the second coupling portion are slidable from basic positions thereof, which face each other with a central axis of the handle body in the longitudinal direction therebetween, toward a proximal end side along a circumferential direction of the rotary plate.

In an existing bi-direction catheter handle, when a rotary plate is rotated to pull one of operation wires toward the proximal end side, the other operation wire in the rotational operation unit may become loosened, and thereby a kink or a breakage of the operation wire may occur.

In contrast, with a catheter handle having the structure described above, when the rotary plate is rotated to pull one of operation wires (the first operation wire or the second operation wire) toward the proximal end side, a coupling portion (the second coupling portion or the first coupling portion) to which the proximal end of the other operation wire (the second operation wire or the first operation wire) is coupled slides from the basic position thereof toward the proximal end side along the circumferential direction of the rotary plate, and the other operation wire is pulled in the proximal end direction, and thus it is possible to prevent the occurrence of loosening of the other operation wire in the rotational operation unit. As a result, it is possible to prevent a kink or a breakage of the operation wire due to loosening of the operation wire.

(7) A distal-end-deflectable catheter according to the present invention includes the catheter shaft and the catheter handle according to the present invention.

The tube component that extends out from a proximal end opening of a lumen that is formed in a semi-circumferential part of the catheter shaft on a side on which the first handle member is positioned is inserted to the inside of the handle body through the guide path formed in the first spacer member.

The tube component that extends out from a proximal end opening of a lumen that is formed in a semi-circumferential part of the catheter shaft on a side on which the second handle member is positioned is inserted to the inside of the handle body through the guide path formed in the second spacer member.

With the distal-end-deflectable catheter having such a structure, the tube component that extends out from a lumen that is formed in the semi-circumferential part of the catheter shaft on a side on which the first handle member is positioned, that is, a lumen that opens on one side of the rotary plate is inserted to the inside of the handle body through the guide path formed in the first spacer member disposed on the one side of the rotary plate, the tube component that extends out from a lumen that is formed in a semi-circumferential part of the catheter shaft on a side on which the second handle member is positioned, that is, a lumen that opens on the other side of the rotary plate is inserted to the inside of the handle body through the guide path formed in the second spacer member disposed on the other side of the rotary plate, and thus the height difference between the lumen opening and the guide path (the distance in the rotation axis direction of the rotary plate) can be reduced, and thus it is possible to prevent a kink of the tube components (accordingly, impairment of flow ability and a breakage of the tube components).

### Advantageous Effects of Invention

A catheter handle according to the present invention can sufficiently elongate the pull amount by which an operation wire is pulled by rotating a rotary plate and can cause a tube component that extends out from a proximal end of a catheter shaft to be inserted to the inside of a handle body without allowing the tube component to contact the rotary plate and the operation wire.

### Brief Description of Drawings

[Fig. 1] Fig. 1 is a plan view illustrating an electrode catheter including a handle according to an embodiment of the present invention.
[Fig. 2] Fig. 2 is a cross-sectional view illustrating a main part of the handle illustrated in Fig. 1 (sectional view taken along line II-II).
[Fig. 3] Fig. 3 is a longitudinal sectional view illustrating a main part of the handle illustrated in Fig. 1 (sectional view taken along line III-III).
[Fig. 4] Fig. 4 is a sectional view illustrating a main part of the handle illustrated in Fig. 1 (sectional view taken along line IV-IV of Fig. 2).
[Fig. 5] Fig. 5 is a sectional view illustrating a main part of the handle illustrated in Fig. 1 (sectional view taken along line V-V of Fig. 2).
[Fig. 6] Fig. 6 is a sectional view of the electrode catheter illustrated in Fig. 1 (sectional view taken along line VI-VI).
[Fig. 7] Fig. 7 is a perspective view illustrating constituent components of the handle illustrated in Fig. 1.
[Fig. 8] Fig. 8 is a plan view illustrating a catheter including an existing handle.
[Fig. 9] Fig. 9 is a cross-sectional view illustrating a main part of the handle illustrated in Fig. 8 (sectional view taken along line IX-IX).
[Fig. 10] Fig. 10 is a sectional view illustrating a main part of the handle illustrated in Fig. 8 (sectional view taken along line X-X).
[Fig. 11] Fig. 11 is a plan view schematically illustrating the inside of an existing handle.

### Description of Embodiments

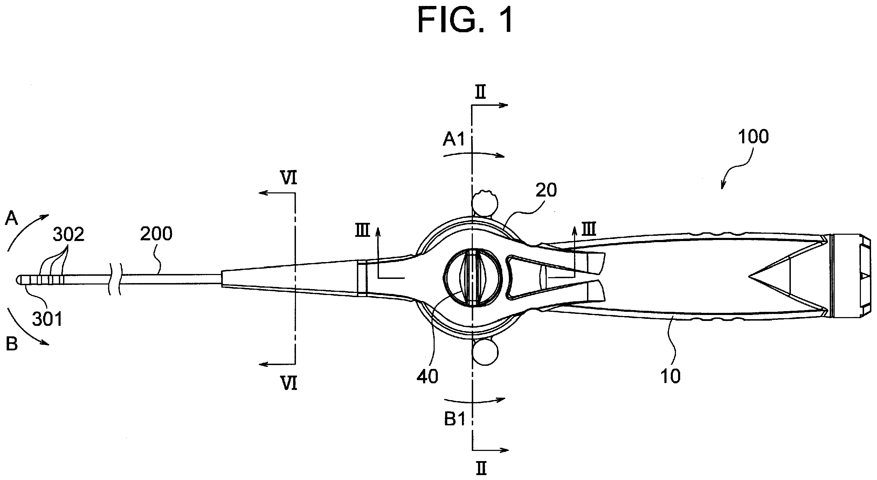

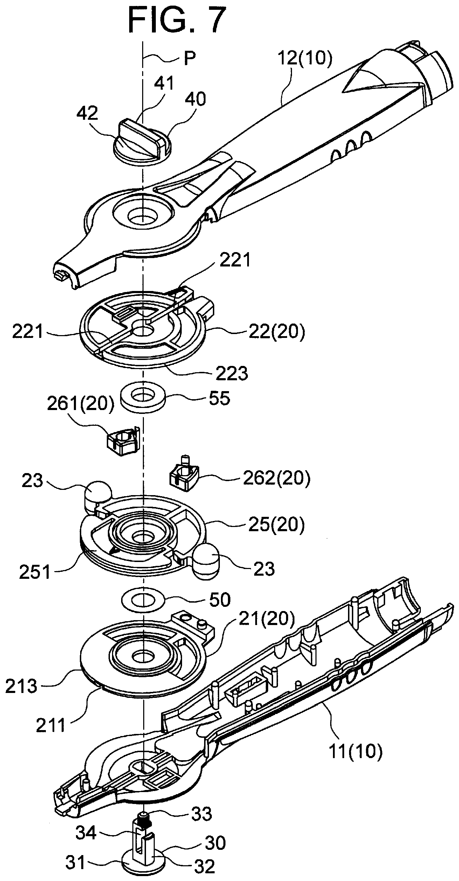

A handle 100 according to the present embodiment illustrated in Figs. 1 to 7 is a handle that is attached to a proximal end side of a catheter shaft 200 having a multi-lumen structure and that constitutes an electrode catheter (distal-end-deflectable catheter). The handle 100 includes: a handle body 10 that is formed by combining a first handle member 11 and a second handle member 12; a rotational operation unit 20 that is disposed between the first handle member 11 and the second handle member 12, that includes a rotary plate 25 that is disc-shaped and that is attached to the handle body 10 so as to be rotatable around a rotation axis (P), and to which each proximal end of a first operation wire 501 and a second operation wire 502 for respectively deflecting a distal end of the catheter shaft 200 in a first direction and in a second direction is coupled; an adjustment pin 30 including a base portion 31 that is fixed to the first handle member 11, a shaft portion 32 that is disposed so as to extend through the rotary plate 25 along the rotation axis (P), and a male thread portion 33 formed on the distal end side of the shaft portion 32; an adjustment knob 40 that includes a female thread portion 43 threadedly engaged with the male thread portion 33 of the adjustment pin 30 and that is rotatably attached to the second handle member 12; an O-ring 55 (elastic member) that is disposed between the second handle member 12 and the rotary plate 25 so that the operational force of the rotary plate 25 changes in accordance with the threaded engagement depth of the male thread portion 33 of the adjustment pin 30 and the female thread portion 43 of the adjustment knob 40; and a washer 50 that is disposed between the first handle member 11 and the rotary plate 25.

The rotational operation unit 20 includes: the rotary plate 25; a first spacer member 21 that is disposed between the rotary plate 25 and the first handle member 11, that is non-rotatably attached to the handle body 10, and that includes an outer peripheral wall 213 having a circular shape coinciding with an outer peripheral shape of the rotary plate 25; a second spacer member 22 that is disposed between the rotary plate 25 and the second handle member 12, that is non-rotatably attached to the handle body 10, and that includes an outer peripheral wall 223 having a circular shape coinciding with the outer peripheral shape of the rotary plate 25; a first coupling portion 261 to which the proximal end of the first operation wire 501 is coupled; and a second coupling portion 262 to which the proximal end of the second operation wire 502 is coupled.

The rotary plate 25 includes a guide rail 251 (guide mechanism) that defines paths of the first operation wire 501 and the second operation wire 502 in the rotational operation unit 20 so that the paths detour along the circumferential direction of the rotary plate 25. In the first spacer member 21, guide grooves 211 (guide paths) for inserting tube components (a flow tube 701 and a protective tube 702), extending out from each proximal end opening of a first lumen 201 and a second lumen 202 of the catheter shaft 200, to the inside of the handle body 10 so that the tube components do not contact the rotary plate 25 are formed. In the second spacer member 22, guide grooves 221 (guide paths) for inserting tube components (a flow tube 703 and a protective tube 704), extending out from each proximal end opening of a third lumen 203 and a fourth lumen 204 of the catheter shaft 200, to the inside of the handle body 10 so that the tube components do not contact the rotary plate 25 are formed. In the shaft portion 32 of the adjustment pin 30, a cutout 34 through which each of the tube components (the flow tube 701 and 703, the protective tube 702 and 704) is insertable is formed.

The catheter illustrated in Fig. 1 is an electrode catheter that is used to diagnose or to treat arrhythmia, and the electrode catheter is composed of the catheter shaft 200 and the handle 100 according to the present embodiment, which is attached to the proximal end side of the catheter shaft 200.

To a distal end part of the catheter shaft 200, a distal end electrode 301 and three ring-shaped electrodes 302 are attached.

The catheter shaft 200 has a multi-lumen structure.

The outside diameter of the catheter shaft 200 is usually 0.6 to 3.0 mm, and the inside diameter of the catheter shaft 200 is usually 0.5 to 2.5 mm.

The distal end electrode 301 and the ring-shaped electrodes 302 are made of, for example, a metal having high electrical conductivity, such as aluminum, copper, stainless steel, gold, or platinum. The outside diameter of the distal end electrode 301 and the ring-shaped electrodes 302 is not particularly limited, but preferably, is approximately the same as the outside diameter of the catheter shaft 200.

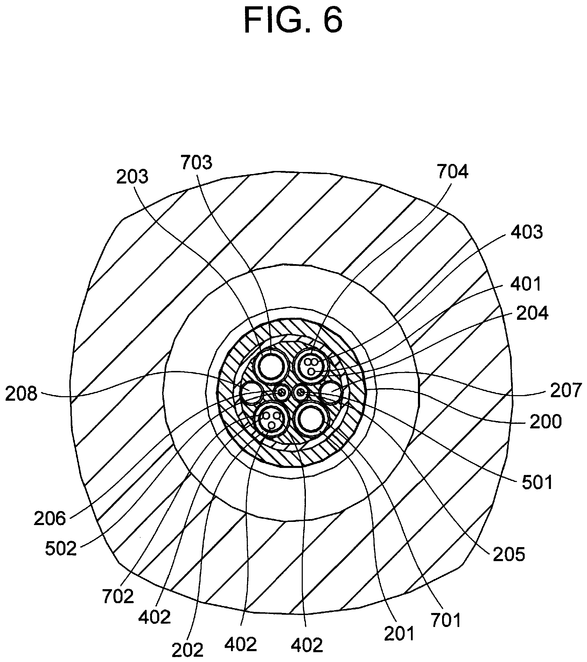

As illustrated in Fig. 6, in the catheter shaft 200, eight lumens 201 to 208 including the following are formed: the first lumen 201 and the second lumen 202 that are formed in a semi-circumferential part (a lower part of the cross section of the shaft 200 illustrated in the figure) of the catheter shaft 200 on a side on which the first handle member 11 is positioned; and the third lumen 203 and the fourth lumen 204 that are formed in a semi-circumferential part (an upper part of the cross section of the shaft 200 illustrated in the figure) of the catheter shaft 200 on a side on which the second handle member 12 is positioned.

In the first lumen 201 and the third lumen 203, respectively, the flow tube 701 and the flow tube 703, through which saline solution for irrigation is to flow, extend.

In the second lumen 202, the protective tube 702, which encloses the conductive wires 402 each of which is connected to a corresponding one of the ring-shaped electrodes 302, extend.

In the fourth lumen 204, the protective tube 704, which encloses a conductive wire 401 connected to the distal end electrode 301 and a thermocouple 403, extends.

In the fifth lumen 205, the first operation wire 501, for deflecting the distal end of the catheter shaft 200 in a first direction, extends.

The first operation wire 501 extends in the seventh lumen 207 in the distal end portion of the catheter shaft 200. The distal end of the first operation wire 501 is connected and fixed to the distal end electrode 301 by using solder with which the inner space of the distal end electrode 301 is filled.

On the other hand, the proximal end of the first operation wire 501 is fixed to the first coupling portion 261 (described below) for coupling the first operation wire 501 to the rotational operation unit 20.

In the sixth lumen 206, the second operation wire 502, for deflecting the distal end of the catheter shaft 200 in a second direction that is a direction opposite to the first direction, extends.

The second operation wire 502 extends in the eighth lumen 208 in the distal end portion of the catheter shaft 200. The distal end of the second operation wire 502 is connected and fixed to the distal end electrode 301 by using solder with which the inner space of the distal end electrode 301 is filled.

On the other hand, the proximal end of the second operation wire 502 is fixed to the second coupling portion 262 (described below) for coupling the second operation wire 502 to the rotational operation unit 20.

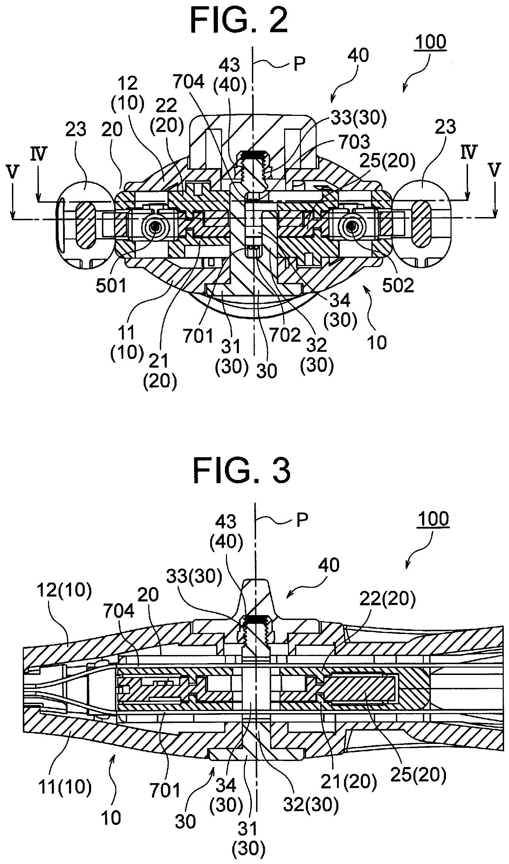

As illustrated in Figs. 2, 3, and 7, the handle body 10 of the handle 100 according to the present embodiment is constituted by combining the first handle member 11 and the second handle member 12 that are separately formed.

The rotational operation unit 20 of the handle 100 according to the present embodiment includes the rotary plate 25 (movable member), the first spacer member 21, the second spacer member 22, the first coupling portion 261, and the second coupling portion 262.

The rotary plate 25 of the rotational operation unit 20 is attached to the handle body 10 so as to be rotatable around the rotation axis (P).

The rotary plate 25 includes the guide rail 251 (guide mechanism) that defines the paths of the first operation wire 501 and the second operation wire 502 in the rotational operation unit 20 so that the paths detour along the circumferential direction of the rotary plate 25. Knobs 23, 23 are provided in the rotary plate 25.

When the rotary plate 25 is rotated in the arrow A1 direction shown in Fig. 1, the first operation wire 501 is pulled, and thus the distal end portion of the catheter shaft 200 is bent in the arrow A direction, and the distal end is deflected.

On the other hand, when the rotary plate 25 is rotated in the arrow B1 direction shown in the figure, the second operation wire 502 is pulled, and thus the distal end portion of the catheter shaft 200 is bent in the arrow B direction and the distal end is deflected.

The first spacer member 21 is disposed on one side of the rotary plate 25, and the second spacer member 22 is disposed on the other side of the rotary plate 25. Here, the first spacer member 21 and the second spacer member 22 are members having the same shape.

The first spacer member 21 of the rotational operation unit 20 is non-rotatably attached to the handle body 10 between the rotary plate 25 and the first handle member 11.

Because the first spacer member 21 is non-rotatable relative to the handle body 10, even when the rotary plate 25 is rotated relative to the handle body 10, the first spacer member 21 does not rotate together with this.

The first spacer member 21 is formed by integrally forming the outer peripheral wall 213, which has a shape coinciding with the outer peripheral shape of the rotary plate 25, and the guide groove 211, which extends in the longitudinal direction of the handle body 10.

The outer peripheral wall 213 of the first spacer member 21 has a shape coinciding with the outer peripheral shape of the rotary plate 25, that is, a generally annular shape (partial annular shape) having substantially the same outside diameter as the rotary plate 25.

The guide groove 211 of the first spacer member 21 is a guide path for inserting, to the inside of the handle body 10, the flow tube 701, which extends out from the proximal end opening of the first lumen 201 of the catheter shaft 200, and the protective tube 702, which extends out from the proximal end opening of the second lumen 202.

The guide groove 211 has a depression (bottom) on a side on which the rotary plate 25 is positioned.

However, the bottom surface of the guide groove 211 is separated from one surface of the rotary plate 25 in the rotation axis (P) direction, and the flow tube 701 and the protective tube 702, which are inserted through the guide groove 211, do not contact the rotary plate 25.

With the first spacer member 21, it is possible to reliably provide a space for inserting the flow tube 701 and the protective tube 702 between the rotary plate 25 and the first handle member 11. By passing the flow tube 701 and the protective tube 702 through the guide groove 211 of the first spacer member 21, it is possible to insert these tube components to the inside of the handle body 10 without allowing the tube components to contact the rotary plate 25 and the operation wires (the first operation wire 501 and the second operation wire 502). Thus, it is possible to reliably prevent generation of a noise and damage to (abrasion of) the flow tube 701 and the protective tube 702 during the operation of the rotary plate 25.

The second spacer member 22 of the rotational operation unit 20 is non-rotatably attached to the handle body 10 between the rotary plate 25 and the second handle member 12.

Because the first spacer member 22 is non-rotatable relative to the handle body 10, even when the rotary plate 25 is rotated relative to the handle body 10, the first spacer member 22 does not rotate together with this.

The second spacer member 22 is formed by integrally forming the outer peripheral wall 223, which has a shape coinciding with the outer peripheral shape of the rotary plate 25, and the guide groove 221, which extends in the longitudinal direction of the handle body 10.

The outer peripheral wall 223 of the second spacer member 22 has a shape coinciding with the outer peripheral shape of the rotary plate 25, that is, a generally annular shape (partial annular shape) having substantially the same outside diameter as the rotary plate 25.

The guide groove 221 of the second spacer member 22 is a guide path for inserting, to the inside of the handle body 10, the flow tube 703, which extends out from the proximal end opening of the third lumen 203 of the catheter shaft 200, and the protective tube 704, which extends out from the proximal end opening of the fourth lumen 204.

The guide groove 221 has a depression (bottom) on a side on which the rotary plate 25 is positioned.

However, the bottom surface of the guide groove 221 is separated from one surface of the rotary plate 25 in the rotation axis (P) direction, and the flow tube 703 and the protective tube 704, which are inserted through the guide groove 221, do not contact the rotary plate 25.

With the second spacer member 22, it is possible to reliably provide a space for inserting the flow tube 703 and the protective tube 704 between the rotary plate 25 and the second handle member 12. By passing the flow tube 703 and the protective tube 704 through the guide groove 221 of the second spacer member 22, it is possible to insert these tube components to the inside of the handle body 10 without allowing the tube components to contact the rotary plate 25 and the operation wires (the first operation wire 501 and the second operation wire 502). Thus, it is possible to reliably prevent generation of a noise and damage to (abrasion of) the flow tube 703 and the protective tube 704 during the operation of the rotary plate 25.

The proximal end of the first operation wire 501 is fixed to the first coupling portion 261 of the rotational operation unit 20.

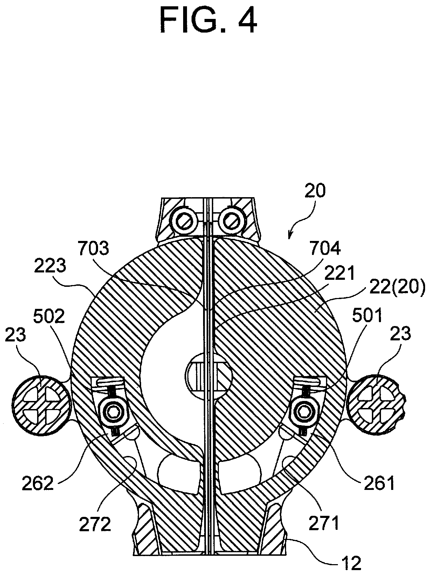

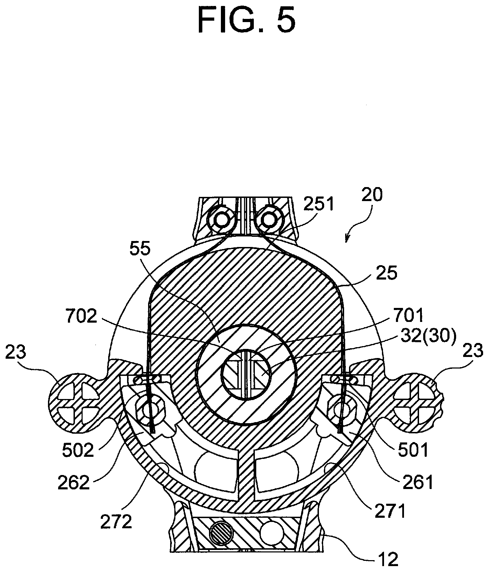

The first coupling portion 261 can be slid along a guideway 271, which is formed along the circumferential direction of the rotary plate 25, toward the proximal end side from the basic position (the position of the first coupling portion 261 when the distal end portion of the catheter shaft is not bent, the position of the first coupling portion 261 illustrated in Figs. 4 and 5).

With such a structure, when the rotary plate 25 is rotated to pull the second operation wire 502, the first coupling portion 261, to which the proximal end of the first operation wire 501 is coupled, slides in the proximal end direction along the circumferential direction of the rotary plate 25, and the first operation wire 501 is pulled in the proximal end direction, and thus it is possible to prevent occurrence of loosening of the first operation wire 501 in the rotational operation unit 20.

The proximal end of the second operation wire 502 is fixed to the second coupling portion 262 of the rotational operation unit 20.

The second coupling portion 262 can be slid along a guideway 272, which is formed along the circumferential direction of the rotary plate 25, toward the proximal end side from the basic position (the position of the second coupling portion 262 when the distal end portion of the catheter shaft is not bent, the position of the second coupling portion 262 illustrated in Figs. 4 and 5).

With such a structure, when the rotary plate 25 is rotated to pull the first operation wire 501, the second coupling portion 262, to which the proximal end of the second operation wire 502 is coupled, slides in the proximal end direction along the circumferential direction of the rotary plate 25, and the second operation wire 502 is pulled in the proximal end direction, and thus it is possible to prevent occurrence of loosening of the second operation wire 502 in the rotational operation unit 20.

As illustrated in Figs. 2, 3, and 7, the adjustment pin 30 of the handle 100 according to the present embodiment is formed by integrally forming the base portion 31, the shaft portion 32, and the male thread portion 33 (distal end part) .

The base portion 31 of the adjustment pin 30 is fixed to the first handle member 11, and the adjustment pin 30 is disposed so as to extend through the rotary plate 25 along the rotation axis (P).

The adjustment knob 40 of the handle 100 according to the present embodiment is formed by integrally forming a knob portion 41, a disc portion 42, and the female thread portion 43.

The female thread portion 43 of the adjustment knob 40 is threadedly engageable with the male thread portion 33 of the adjustment pin 30, and the distance between the first handle member 11 and the second handle member 12 changes in accordance with the threaded engagement depth (screwing depth) of the female thread portion 43 of the adjustment knob 40 and the male thread portion 33 of the adjustment pin 30.

Because the distance changes, a compressive force applied to the O-ring 55, which is disposed between the second spacer member 22 and the rotary plate 25, changes, and a fastening force that the O-ring 55 applies to the rotary plate 25 (operational force of the rotary plate) changes. Here, the operation of the rotary plate 25 becomes heavier when the fastening force applied by the O-ring 55 is increased, and the operation of the rotary plate 25 becomes lighter when the fastening force is reduced.

Here, the O-ring 55 is made of a material having high elasticity, such as silicone rubber, nitrile rubber, or fluorocarbon rubber.

The washer 50, which is disposed between the first spacer member 21 and the rotary plate 25, is preferably made of a material having good sliding characteristics, and is made of, for example, a fluorocarbon resin.

In the shaft portion 32 of the adjustment pin 30, the cutout 34, through which each of the tube components (the flow tubes 701 and 703, the protective tubes 702 and 704) is insertable, is formed.

Thus, it is not necessary to detour each of these tube components around the shaft portion 32 of the adjustment pin 30, and it is possible to cause each of the tube components to extend to the inside of the handle body 10 along the longitudinal direction of the handle body 10.

With the handle 100 according to the present embodiment, it is possible to cause the tube components (the flow tube 701, the protective tube 702, the flow tube 703, and the protective tube 704), which respectively extend out from the proximal end openings of the first lumen 201, the second lumen 202, the third lumen 203, and the fourth lumen 204 of the catheter shaft 200, to extend along the longitudinal direction of the handle body 10.

Moreover, because the rotary plate 25 of the handle 100 includes the guide rail 251 (guide mechanism) that defines the paths of the first operation wire 501 and the second operation wire 502 in the rotational operation unit 20 so that the paths detour along the circumferential direction of the rotary plate 25, it is possible to sufficiently elongate the pull amount by which the first operation wire 501 and the second operation wire 502 are pulled by rotating the rotary plate 25.

Moreover, by passing the flow tube 701 and the protective tube 702 through the guide groove 211 formed in the first spacer member 21, it is possible to insert these tube components to the inside of the handle body 10 without allowing the tube components to contact the rotary plate 25, and, by passing the flow tube 703 and the protective tube 704 through the guide groove 221 formed in the second spacer member 22, it is possible to insert these tube components to the inside of the handle body 10 without allowing the tube components to contact the rotary plate 25.

Because contact of the tube components with the rotary plate 25 can be avoided, it is possible to reliably prevent generation of noise, damage to (abrasion of) the tube components, and the like during the operation of the rotary plate 25.

Furthermore, by passing the tube components (the flow tube 701 and the protective tube 702), respectively extending out from the proximal end openings of the lumens 201 and 202 of the catheter shaft 200, through the guide groove 211 of the first spacer member 21, and by passing the tube components (the flow tube 703 and the protective tube 704), respectively extending out from the proximal end openings of the lumens 203 and 204, through the guide groove 221 of the second spacer member 22, it is possible to insert these tube components to the inside of the handle body without allowing the tube components to contact the operation wires (the first operation wire 501 and the second operation wire 502).

Heretofore, embodiments of the present invention have been described. However, a catheter handle according to the present invention is not limited to these, and can be modified in various ways.

For example, the number of tube components that are respectively inserted through the guide groove 211 and the guide groove 221 is not limited to two, and may be one, or may be three or more.

In the embodiment described above, an example in which a catheter handle according to the present invention is applied to an electrode catheter has been described. However, a catheter handle according to the present invention can be applied also to a guide catheter (guiding catheter), an angiographic catheter, a sheath catheter (sheath introducer), a microcatheter, and the like. Reference Signs List

- 100: handle
- 10: handle body
- 11: first handle member
- 12: second handle member
- 20: rotational operation unit
- 21: first spacer member
- 211: guide groove (guide path)
- 213: outer peripheral wall
- 22: second spacer member
- 221: guide groove (guide path)
- 223: outer peripheral wall
- 23: knob
- 25: rotary plate
- 251: guide rail (guide mechanism)
- 261: first coupling portion
- 262: second coupling portion
- 30: adjustment pin
- 31: base portion
- 32: shaft portion
- 33: male thread portion
- 34: cutout
- 40: adjustment knob
- 41: knob portion
- 42: disc portion
- 43: female thread portion
- 50: washer
- 55: O-ring
- 200: catheter shaft
- 201: first lumen
- 202: second lumen
- 203: third lumen
- 204: fourth lumen
- 205: fifth lumen
- 206: sixth lumen
- 207: seventh lumen
- 208: eighth lumen
- 301: distal end electrode
- 302: ring-shaped electrode
- 401: conductive wire of distal end electrode
- 402: conductive wire of ring-shaped electrode
- 403: thermocouple
- 501: first operation wire
- 502: second operation wire
- 701,703: flow tube (tube component)
- 702,704: protective tube (tube component)

## Claims

1. A catheter handle to be attached to a proximal end side of a catheter shaft, comprising:
a handle body that is formed by combining a first handle member and a second handle member that are divided into two along a longitudinal direction; and
a rotational operation unit that is disposed between the first handle member and the second handle member, that includes a rotary plate that is disc-shaped and that is attached to the handle body so as to be rotatable around a rotation axis perpendicular to the longitudinal direction, and to which each proximal end of a first operation wire and a second operation wire for respectively deflecting a distal end of the catheter shaft in a first direction and in a second direction is coupled,
wherein the rotational operation unit includes the rotary plate,
a first spacer member that is disposed between the rotary plate and the first handle member, that is non-rotatably attached to the handle body, and that includes an outer peripheral wall having a shape coinciding with an outer peripheral shape of the rotary plate, and
a second spacer member that is disposed between the rotary plate and the second handle member, that is non-rotatably attached to the handle body, and that includes an outer peripheral wall having a shape coinciding with the outer peripheral shape of the rotary plate,
wherein the rotary plate includes a guide mechanism that defines paths of the first operation wire and the second operation wire in the rotational operation unit so that the paths detour along a circumferential direction of the rotary plate, and
wherein, in the first spacer member and the second spacer member, respectively, guide paths for inserting tube components extending out from a proximal end of the catheter shaft to an inside of the handle body so that the tube components do not contact the rotary plate are formed.

2. The catheter handle according to Claim 1, comprising:
an adjustment pin including a base portion that is fixed to the first handle member, a shaft portion that is disposed so as to extend through the rotary plate along the rotation axis of the rotary plate, and a male thread portion that is formed on a distal end side of the shaft portion;
an adjustment knob that includes a female thread portion threadedly engaged with the male thread portion of the adjustment pin and that is rotatably attached to the second handle member; and
an elastic member that is disposed between the handle body and the rotary plate so that an operational force of the rotary plate changes in accordance with a threaded engagement depth of the male thread portion of the adjustment pin and the female thread portion of the adjustment knob,
wherein, in the shaft portion of the adjustment pin, a through-hole or a cutout through which each of the tube components is insertable is formed.

3. The catheter handle according to Claim 1 or 2, wherein the guide paths that are respectively formed in the first spacer member and the second spacer member are guide grooves that are integrally formed with the outer peripheral walls and that have depressions on the rotary plate side.

4. The catheter handle according to any one of Claims 1 to 3, wherein at least one of the tube components is a flow tube through which a fluid is to flow.

5. The catheter handle according to any one of Claims 1 to 3, wherein at least one of the tube components is a protective tube that encloses a conductive wire and/or another component.

6. The catheter handle according to any one of Claims 1 to 5, wherein the rotational operation unit includes a first coupling portion to which the proximal end of the first operation wire is coupled and a second coupling portion to which the proximal end of the second operation wire is coupled, and
wherein the first coupling portion and the second coupling portion are slidable from basic positions thereof, which face each other with a central axis of the handle body in the longitudinal direction therebetween, toward a proximal end side along a circumferential direction of the rotary plate.

7. A distal-end-deflectable catheter comprising:
the catheter shaft and the catheter handle according to any one of Claims 1 to 6,
wherein the tube component that extends out from a proximal end opening of a lumen that is formed in a semi-circumferential part of the catheter shaft on a side on which the first handle member is positioned is inserted to the inside of the handle body through the guide path formed in the first spacer member, and
wherein the tube component that extends out from a proximal end opening of a lumen that is formed in a semi-circumferential part of the catheter shaft on a side on which the second handle member is positioned is inserted to the inside of the handle body through the guide path formed in the second spacer member.
